# EUROPEAN PATENT APPLICATION

(11) **EP 4 613 272 A1**
(43) Date of publication of application: **10.09.2025**
(21) Application number: 23885716.3
(22) Date of filing: 30.10.2023
(51) Int. Cl.: A61K 31/496, A61P 25/00

(54) **USE OF PYRIDINE DERIVATIVE WHICH IS TRPV1 ANTAGONIST**

(30) Priority: 31.10.2022 JP 2022174020
(71) Applicant: Shionogi & Co., Ltd, Osaka-shi, Osaka 541-0045 (JP)
(72) Inventor: YAMAKAWA, Hidekuni, Osaka-shi, Osaka 541-0045 (JP)
(74) Representative: Vossius & Partner Patentanwälte Rechtsanwälte mbB
(86) International application number: PCT/JP2023/039098
(87) International publication number: WO 2024/095962

(57) **Abstract**

The present invention provides a new pharmaceutical composition and a method for treating and/or preventing social impairments such as autism spectrum disorder, fragile X syndrome, and/or autism spectrum disorder-like symptoms. Specifically, provided is a pharmaceutical composition for the treatment and/or prevention of social impairment, including a compound represented by Formula (I): 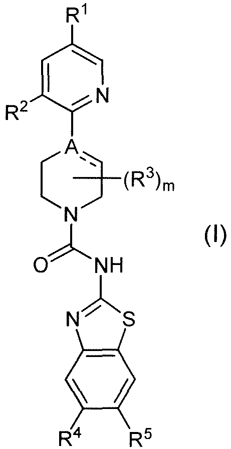 wherein R¹ is substituted or unsubstituted alkyl, R² is a hydrogen atom or the like, A is N or the like, dashed lines indicate presence or absence of binding, R³ is substituted or unsubstituted alkyl or the like, m is 0 or 1, R⁴ and R⁵ are each independently a hydrogen atom or the like, or a pharmaceutically acceptable complex thereof.

## Description

### [TECHNICAL FIELD]

The present invention relates to new pharmaceutical compositions and methods for the treatment and/or prevention of social impairments, such as autism spectrum disorder, fragile X syndrome, autism spectrum disorder-like symptoms and/or disorders in social communication and/or reciprocal social interaction, particularly preferably for the treatment of autism spectrum disorder.

### [BACKGROUND ART]

Autism spectrum disorder (ASD) is a disease characterized by the presence of persistent disorders in social communication and reciprocal social interaction in multiple contexts, the presence of two or more restrictive and repetitive patterns of behaviors, interests, and activities (emotional, repetitive body movements and conversations, persistence and obsessiveness, extremely limited and preoccupied interest, hypersensitivity or obtundation to sensory stimuli, etc.), and the presence of these symptoms from early development. There are 3.8 million patients in the world (Non-patent Document 1) and 350,000 or more patients in Japan, and it is said that the economic burden required per patient is 5 million yen or more per year (Non-patent Document 2), and in many cases, considering that the disease period is a lifetime, the economic influence is very large. However, there is currently no effective treatment agent for the core symptoms of ASD. As a treatment for ASD, behavior therapy such as applied behavior analysis is central, but since behavior therapy requires a lot of time and human resources, and it is known that the effect decreases as the age increases, patients who can enjoy the benefit are very limited. From the above background, there is a strong demand for a treatment agent effective for core symptoms of ASD.

At present, as a target molecule of an ASD treatment agent, for example, a molecule known to be associated with social behavior such as oxytocin or vasopressin and a receptor thereof are attracting attention.

In 2016, a group of Ru-Rong Ji et al. reported that the function of TRPV1 was reduced due to the deficiency of SHANK3, which is one of the causative genes of genetic ASD (Non-patent Document 3). Many of SHANK3 gene mutations observed in ASD patients are mutations that impair or reduce the function, and considering that SHANK3-deficient mice exhibit ASD-like symptoms, it is inferred that the function of the TRPV1 receptor may be reduced in at least some ASD patients. However, there are still no pharmacological reports indicating an association between TRPV1 receptor and ASD.

It is known that patients with human fragile X syndrome (FXS), which is caused by abnormalities of the X chromosome, present with social impairment symptoms, such as those often seen in ASD (Non-patent Documents 4 to 6).

Non-patent Document 7 and Patent Literatures 1 to 6 describe TRPV1 (transient receptor potential vanilloid type 1) antagonists, but do not describe or suggest social impairments and ASD.

### [PRIOR ART REFERENCES]

### [Patent Document]

[Patent Document 1] International Publication WO2005/009988A
[Patent Document 2] International Publication WO2008/132600A
[Patent Document 3] International Publication WO2011/162409A
[Patent Document 4] International Publication WO2012/158844A
[Patent Document 5] International Publication WO2012/176061A
[Patent Document 6] US 2020/0197378A1

### [Non-patent Document]

[Non-patent Document 1] JAMA Pediatr. 2014; 168(8): 721-728.
[Non-patent Document 2] Pediatrics 2014; 133: e520-e529
[Non-patent Document 3] Neuron. 2016 Dec 21; 92(6): 1279-1293.
[Non-patent Document 4] A neurochemical theory of autism. Trends Neurosci 2: 174-177.
[Non-patent Document 5] J Intellect Disabil Res. 2015 Apr; 59(4): 293-306.
[Non-patent Document 6] Br J Pharmacol. 2018 Jul; 175(14): 2750-2769.
[Non-patent Document 7] Biochemical Pharmacology 78 (2009) 211-216.

### [SUMMARY OF THE INVENTION]

### [PROBLEMS TO BE SOLVED BY THE INVENTION]

Provided are new pharmaceutical compositions and methods for the treatment and/or prevention of social impairments, such as autism spectrum disorder, fragile X syndrome, autism spectrum disorder-like symptoms and/or disorders in social communication and/or reciprocal social interaction, particularly preferably for the treatment of autism spectrum disorder.

### [MEANS FOR SOLVING THE PROBLEM]

The present inventor has extensively studied pharmaceutical compositions and methods for treating and/or preventing social impairments, such as autism spectrum disorder, fragile X syndrome, autism spectrum disorder-like symptoms, and/or disorders in social communication and/or reciprocal social interaction, particularly preferably for treating autism spectrum disorder, and has found suitable active ingredients.

The present invention relates to, for example, the following.
(1) A pharmaceutical composition for treatment and/or prevention of social impairment, comprising a compound represented by Formula (I): 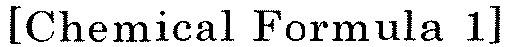 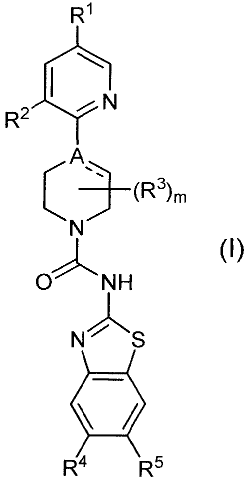 wherein
   R¹ is substituted or unsubstituted alkyl,
   R² is a hydrogen atom, halogen, or substituted or unsubstituted alkyl,
   A is N, CH, or C,
   when A is N or CH, a dashed line indicates absence of a bond,
   when A is C, the dashed line indicates presence of a bond,
   R³ is substituted or unsubstituted alkyl or halogen,
   m is 0 or 1, and
   R⁴ and R⁵ are each independently a hydrogen atom, halogen, substituted or unsubstituted alkyl, substituted or unsubstituted alkyloxy, or substituted or unsubstituted alkyloxycarbonyl,
   or a pharmaceutically acceptable complex thereof.
(2) The pharmaceutical composition according to (1) above, wherein
   R¹ is C2-C3 alkyl substituted with one or more hydroxy,
   R² is halogen, 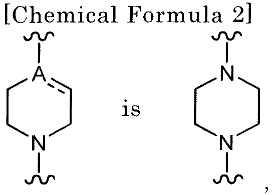
   R³ is methyl, and
   R⁴ and R⁵ are each independently a hydrogen atom, halogen, or methyl.
(3) The pharmaceutical composition according to according to (1) or (2) above, wherein the social impairment is a disorder in social communication and/or reciprocal social interaction.
(4) The pharmaceutical composition according to any one of (1) to (3) above, wherein the social impairment is autism spectrum disorder, fragile X syndrome, and/or autism spectrum disorder-like symptoms.
(5) The pharmaceutical composition according to any one of (1) to (4) above, wherein the social impairment is autism spectrum disorder.
(6) The pharmaceutical composition according to any one of (1) to (5) above, wherein
   the compound represented by the Formula (I) or a pharmaceutically acceptable complex thereof is the following compound: 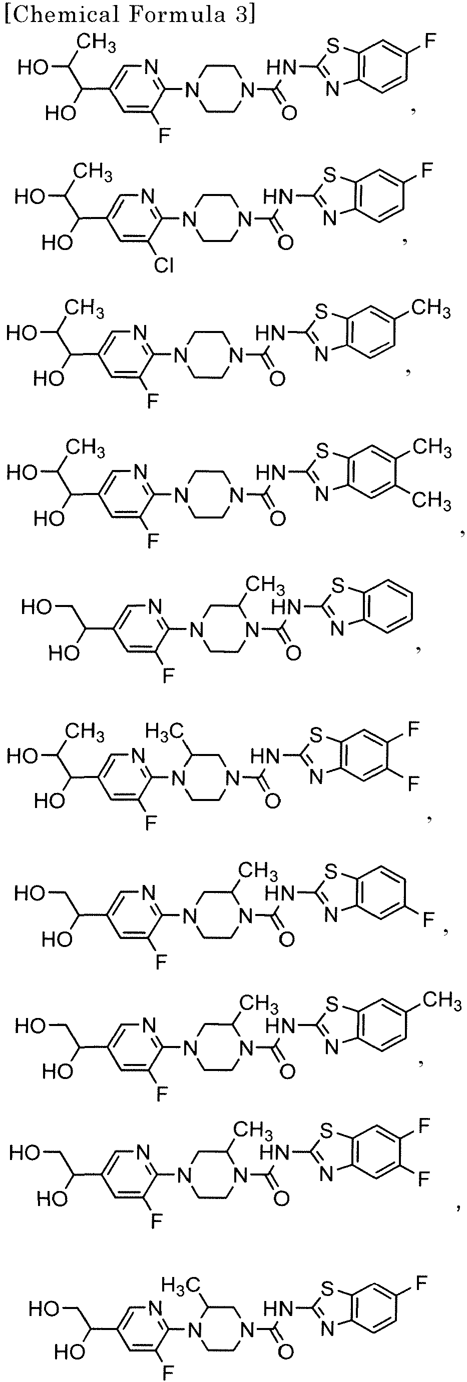 or
   or a pharmaceutically acceptable complex thereof.
(6A) The pharmaceutical composition according to any one of (1) to (6) above, wherein
   the compound represented by the Formula (I) or a pharmaceutically acceptable complex thereof is the following compound: 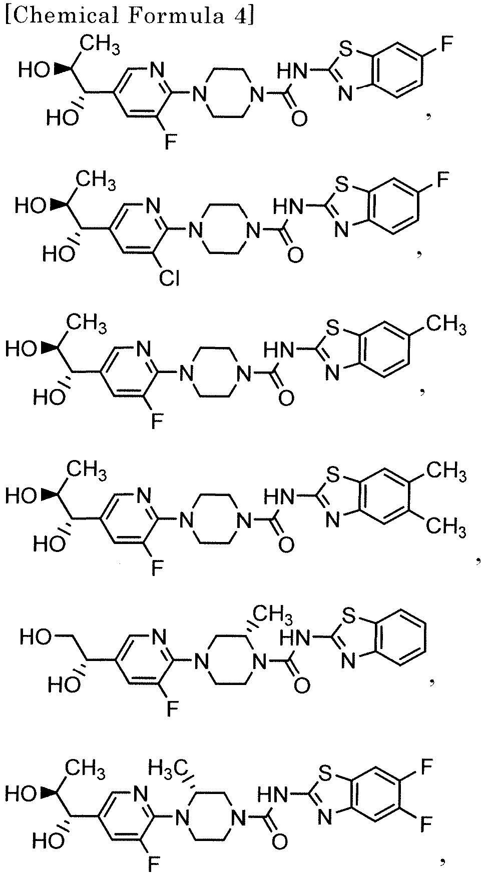 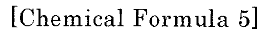 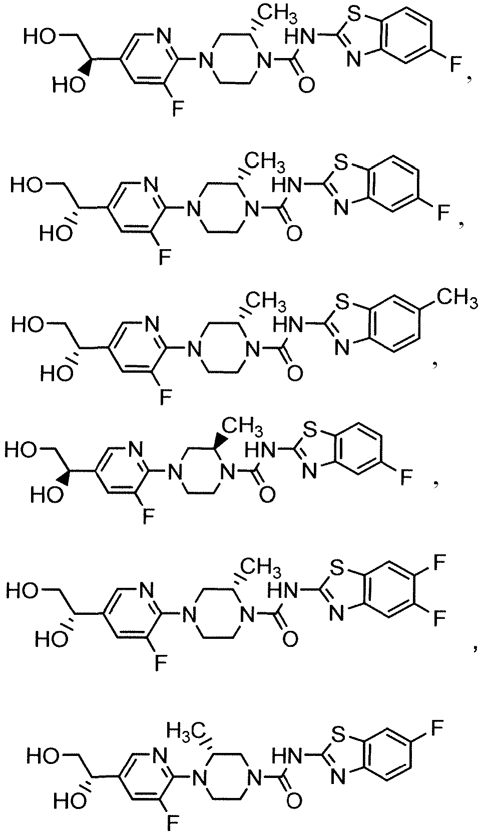 or
   or a pharmaceutically acceptable complex thereof.
(7) The pharmaceutical composition according to any one of (1) to (6) above, wherein
   the compound represented by the Formula (I) or a pharmaceutically acceptable complex thereof is a compound represented by Formula (I-A): 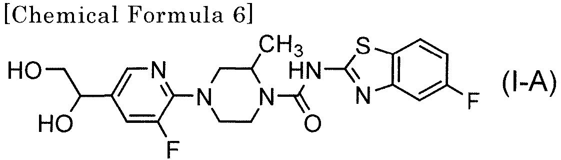
   or a pharmaceutically acceptable complex thereof.
(8) The pharmaceutical composition according to any one of (1) to (7) above, wherein
   the compound represented by the Formula (I) or a pharmaceutically acceptable complex thereof is a compound represented by Formula (I-B): 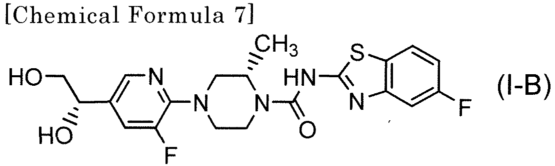
   or a pharmaceutically acceptable complex thereof.
(9) The pharmaceutical composition according to any one of (1) to (8) and (6A) above, wherein the pharmaceutically acceptable complex is a complex with hydrochloric acid, tartaric acid, benzenesulfonic acid, p-toluenesulfonic acid, or fumaric acid.
(10) The pharmaceutical composition according to any one of (1) to (9) and (6A) above, wherein the pharmaceutically acceptable complex is a complex with fumaric acid.
(11) The pharmaceutical composition according to any one of (1) to (10) and (6A) above, wherein the pharmaceutically acceptable complex is a fumaric acid cocrystal.
(12) The pharmaceutical composition according to any one of (1) to (11) and (6A) above, wherein the pharmaceutically acceptable complex is a complex in which the molar ratio of the compound represented by the Formula (I) and fumaric acid is about 1 : about 0.5 (or about 2 : about 1).
(13) The pharmaceutical composition according to any one of (1) to (12) and (6A) above, wherein the pharmaceutically acceptable complex is a complex in which the molar ratio of the compound represented by the Formula (I) and fumaric acid is 1 : 0.5 ± 0.2.
(14) The pharmaceutical composition according to any one of (1) to (13) and (6A) above, wherein a dose per one time of the compound represented by the Formula (I) or the pharmaceutically acceptable complex thereof is 1 mg to 1000 mg.
(15) The pharmaceutical composition according to any one of (1) to (14) and (6A) above, for treating and/or preventing a core symptom of autism spectrum disorder.
(101) A method for the treatment and/or prevention of social impairment, comprising administering to a patient in need thereof a compound represented by the Formula (I): 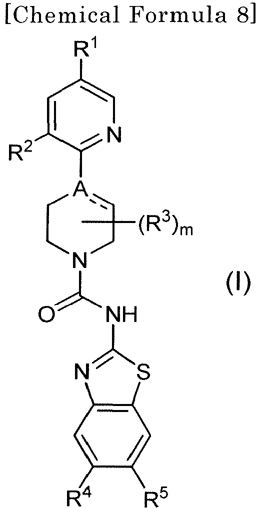 wherein:
   R¹ is substituted or unsubstituted alkyl,
   R² is a hydrogen atom, halogen, or substituted or unsubstituted alkyl,
   A is N, CH, or C,
   when A is N or CH, the dashed line indicates the absence of a bond,
   when A is C, the dashed line indicates the presence of a bond,
   R³ is substituted or unsubstituted alkyl or halogen,
   m is 0 or 1, and
   R⁴ and R⁵ are each independently a hydrogen atom, halogen, substituted or unsubstituted alkyl, substituted or unsubstituted alkyloxy, or substituted or unsubstituted alkyloxycarbonyl,
   or a pharmaceutically acceptable complex thereof.
(102) The method according to (101) above, wherein
   R¹ is C2-C3 alkyl substituted with one or more hydroxy,
   R² is halogen, 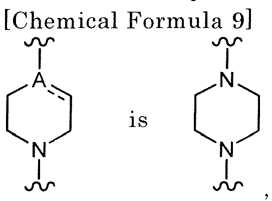
   R³ is methyl, and
   R⁴ and R⁵ are each independently a hydrogen atom, halogen, or methyl.
(103) The method according to (1) or (2) above, wherein the social impairment is a disorder in social communication and/or reciprocal social interaction.
(104) The method according to any one of (101) to (103) above, wherein the social impairment is autism spectrum disorder, fragile X syndrome, and/or autism spectrum disorder-like symptoms.
(105) The method according to any one of (101) to (104) above, wherein the social impairment is autism spectrum disorder.
(106) The method according to any one of (101) to (105) above, wherein
   the compound represented by the Formula (I) or a pharmaceutically acceptable complex thereof is the following compound: 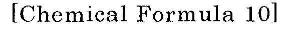 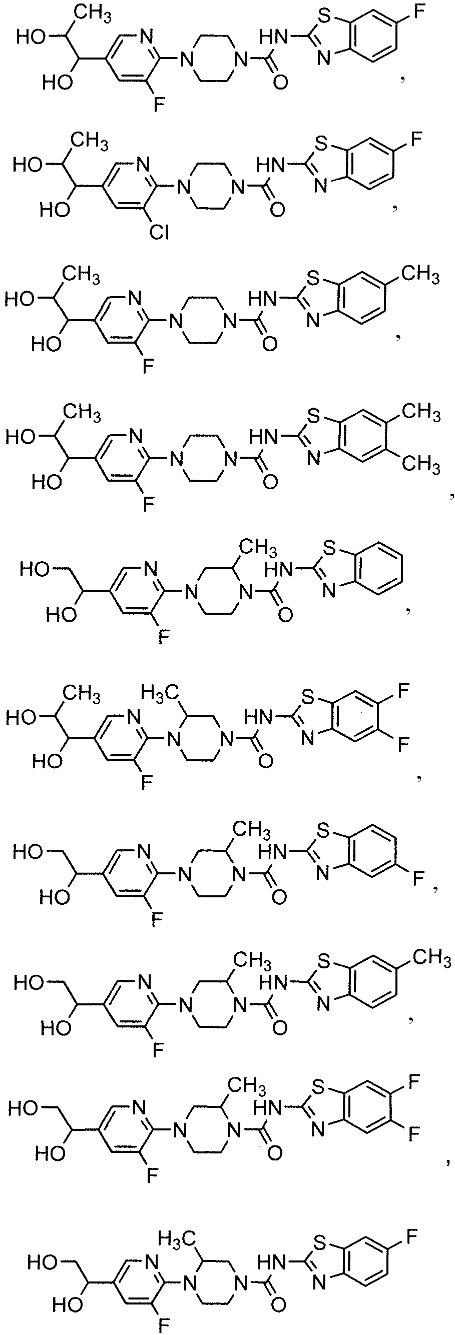 or
   or a pharmaceutically acceptable complex thereof.
(106A) The method according to any one of (101) to (106) above, wherein
   the compound represented by the Formula (I) or a pharmaceutically acceptable complex thereof is the following compound: 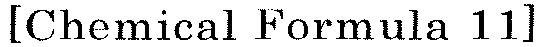 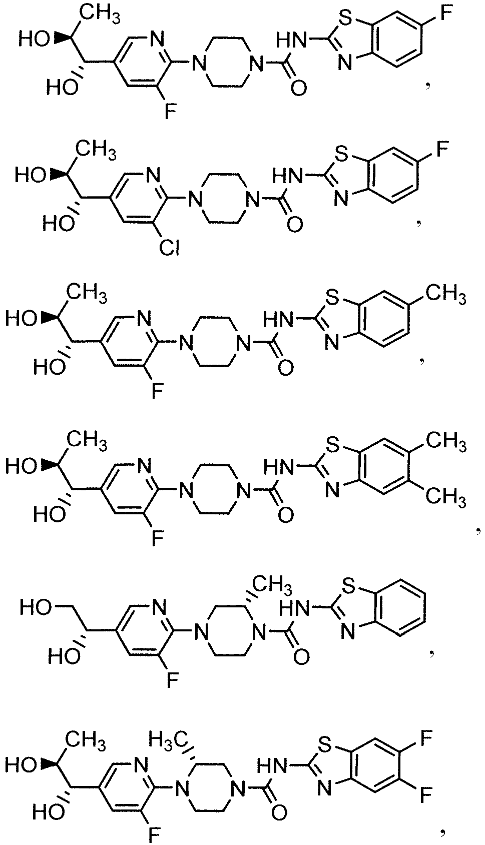 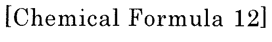 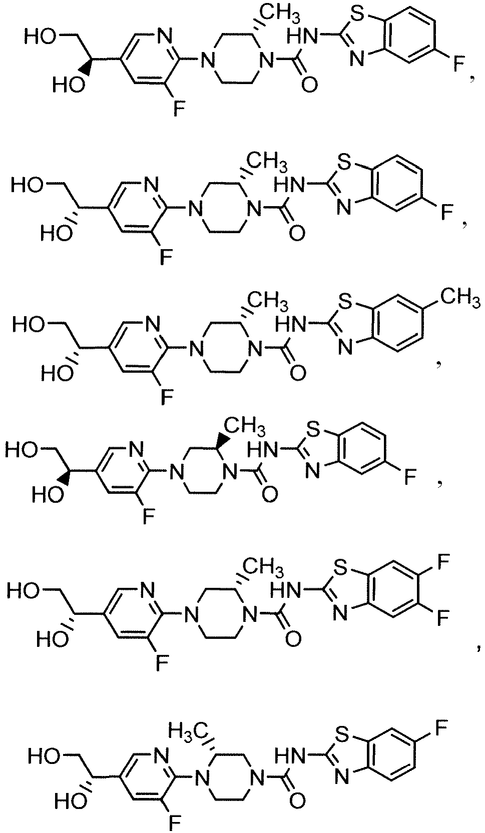 or
   or a pharmaceutically acceptable complex thereof.
(107) The method according to any one of (101) to (106) above, wherein
   the compound represented by the Formula (I) or a pharmaceutically acceptable complex thereof is a compound represented by Formula (I-A): 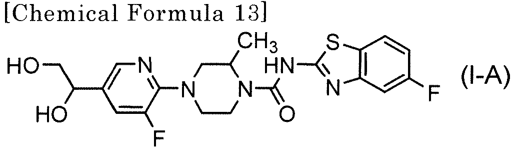
   or a pharmaceutically acceptable complex thereof.
(108) The method according to any one of (101) to (107) above, wherein
   the compound represented by the Formula (I) or a pharmaceutically acceptable complex thereof is a compound represented by Formula (I-B): 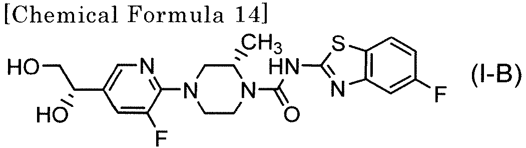
   or a pharmaceutically acceptable complex thereof.
(109) The method according to any one of (101) to (108) and (106A) above, wherein the pharmaceutically acceptable complex is a complex with hydrochloric acid, tartaric acid, benzenesulfonic acid, p-toluenesulfonic acid, or fumaric acid.
(110) The method according to any one of (101) to (109) and (106A) above, wherein the pharmaceutically acceptable complex is a complex with fumaric acid.
(111) The method according to any one of (101) to (110) and (106A) above, wherein the pharmaceutically acceptable complex is a fumaric acid cocrystal.
(112) The method according to any one of (101) to (111) and (106A) above, wherein the pharmaceutically acceptable complex is a complex in which the molar ratio of the compound represented by the Formula (I) and fumaric acid is about 1 : about 0.5 (or about 2 : about 1).
(113) The method according to any one of (101) to (112) and (106A) above, wherein the pharmaceutically acceptable complex is a complex in which the molar ratio of the compound represented by the Formula (I) and fumaric acid is 1 : 0.5 ± 0.2.
(114) The method according to any one of (101) to (113) and (106A) above, wherein a dose per one time of the compound represented by the Formula (I) or the pharmaceutically acceptable complex thereof is 1 mg to 1000 mg.
(115) The method according to any one of (101) to (114) and (106A) above, for treating and/or preventing a core symptom of autism spectrum disorder.
(201) A compound represented by Formula (I): 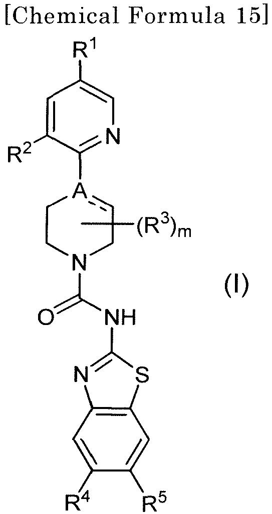 wherein
   R¹ is substituted or unsubstituted alkyl,
   R² is a hydrogen atom, halogen, or substituted or unsubstituted alkyl,
   A is N, CH, or C,
   when A is N or CH, the dashed line indicates the absence of a bond,
   when A is C, the dashed line indicates the presence of a bond,
   R³ is substituted or unsubstituted alkyl or halogen,
   m is 0 or 1, and
   R⁴ and R⁵ are each independently a hydrogen atom, halogen, substituted or unsubstituted alkyl, substituted or unsubstituted alkyloxy, or substituted or unsubstituted alkyloxycarbonyl,
   or a pharmaceutically acceptable complex thereof for use in the treatment and/or prevention of social impairment.
(202) The compound according to (201) above, wherein
   R¹ is C2-C3 alkyl substituted with one or more hydroxy,
   R² is halogen, 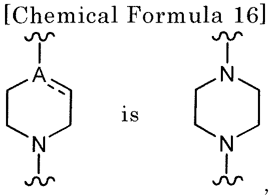
   R³ is methyl, and
   R⁴ and R⁵ are each independently a hydrogen atom, halogen, or methyl,
   or a pharmaceutically acceptable complex thereof.
(203) A compound according to (201) or (202) above, or a pharmaceutically acceptable complex thereof, for use in the treatment and/or prevention of a disorder in social communication and/or reciprocal social interaction.
(204) A compound according to any one of (201) to (203) above or a pharmaceutically acceptable complex thereof, for use in the treatment and/or prevention of autism spectrum disorder, fragile X syndrome, and/or autism spectrum disorder-like symptoms.
(205) A compound according to any one of (201) to (204) above or a pharmaceutically acceptable complex thereof, for use in treatment and/or prevention of autism spectrum disorder.
(206) The compound according to any one of (201) to (205), which is the following compounds: 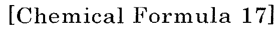 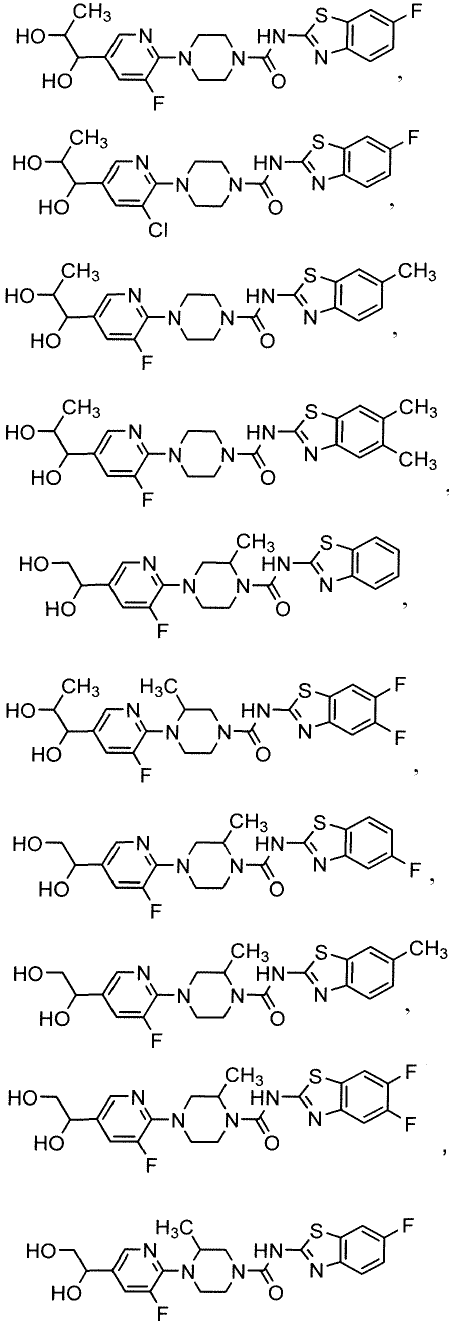 or or a pharmaceutically acceptable complex thereof.
(206A) The compound or a or a pharmaceutically acceptable complex thereof according to any one of (201) to (206), which is the following compounds: 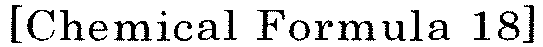 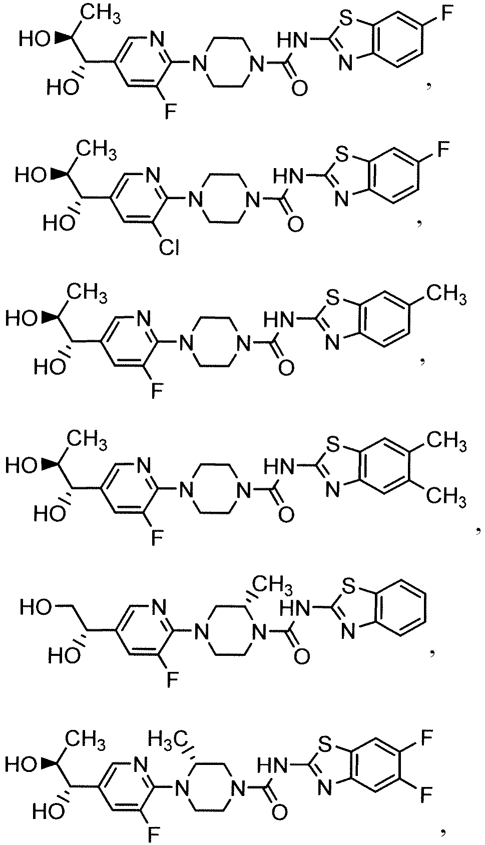 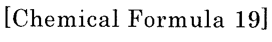 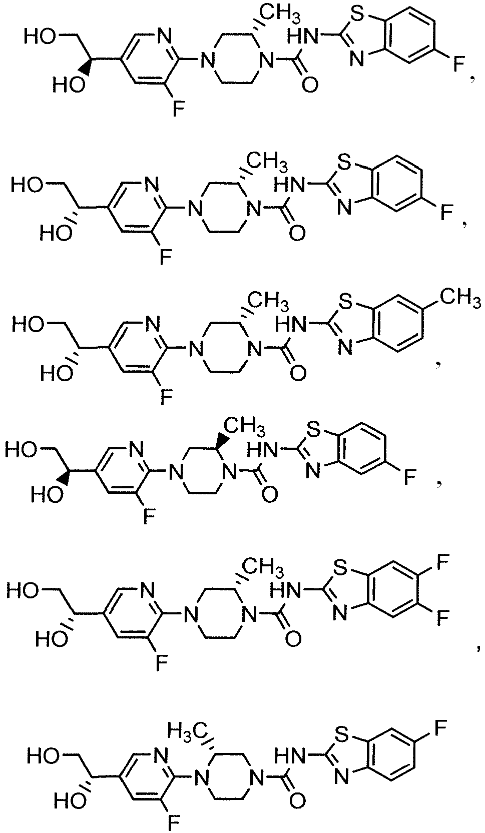 or or a pharmaceutically acceptable complex thereof.
(207) The compound according to any one of (201) to (206) or a pharmaceutically acceptable complex thereof, which is a compound represented by Formula (I-A): 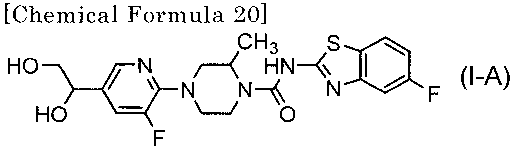 or a pharmaceutically acceptable complex thereof.
(208) The compound according to any one of (201) to (207) or a pharmaceutically acceptable complex thereof, which is a compound represented by Formula (I-B): 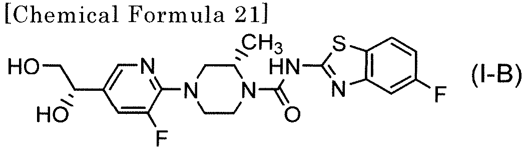 or a pharmaceutically acceptable complex thereof.
(209) The compound according to any one of (201) to (208) and (206A), or a pharmaceutically acceptable complex thereof, wherein the pharmaceutically acceptable complex is a complex with hydrochloric acid, tartaric acid, benzenesulfonic acid, p-toluenesulfonic acid, or fumaric acid.
(210) The compound according to any one of (201) to (209) and (206A), or the pharmaceutically acceptable complex thereof, wherein the pharmaceutically acceptable complex is a complex with fumaric acid.
(211) The compound according to any one of (201) to (210) and (206A) or a pharmaceutically acceptable complex thereof, wherein the pharmaceutically acceptable complex is a fumaric acid cocrystal.
(212) The compound according to any one of (201) to (211) and (206A) above, or a pharmaceutically acceptable complex thereof, wherein the pharmaceutically acceptable complex is a complex in which the molar ratio of the compound represented by the Formula (I) and fumaric acid is about 1 : about 0.5 (or about 2 : about 1).
(213) The compound according to any one of (201) to (212) and (206A) or a pharmaceutically acceptable complex thereof, wherein the pharmaceutically acceptable complex is a complex in which the molar ratio of the compound represented by the Formula (I) and fumaric acid is 1 : 0.5 ± 0.2.
(214) The compound according to any one of (201) to (213) and (206A) or the pharmaceutically acceptable complex thereof, wherein a dose per one time of the compound represented by the Formula (I) or the pharmaceutically acceptable complex thereof is 1 mg to 1000 mg.
(215) A compound according to any one of (201) to (214) and (206A) above or a pharmaceutically acceptable complex thereof, for the treatment and/or prevention of a core symptom of autism spectrum disorder.
(301) Use the compound represented by the Formula (I): 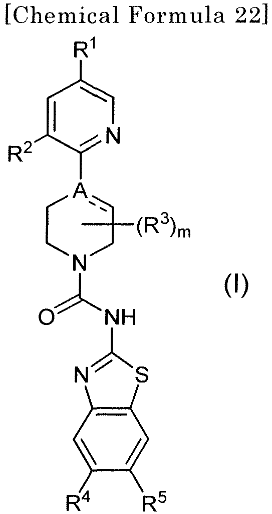
wherein
   R¹ is substituted or unsubstituted alkyl,
   R² is a hydrogen atom, halogen, or substituted or unsubstituted alkyl,
   A is N, CH, or C,
   when A is N or CH, the dashed line indicates the absence of a bond,
   when A is C, the dashed line indicates the presence of a bond,
   R³ is substituted or unsubstituted alkyl or halogen,
   m is 0 or 1, and
   R⁴ and R⁵ are each independently a hydrogen atom, halogen, substituted or unsubstituted alkyl, substituted or unsubstituted alkyloxy, or substituted or unsubstituted alkyloxycarbonyl,
   or a pharmaceutically acceptable complex thereof, for the manufacture of a pharmaceutical composition for the treatment and/or prevention of social impairment.
(302) Use of the compound according to (301) above, wherein
   R¹ is C2-C3 alkyl substituted with one or more hydroxy,
   R² is halogen, 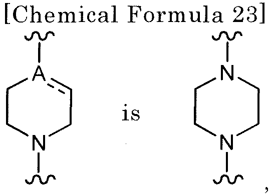
   R³ is methyl, and
   R⁴ and R⁵ are each independently a hydrogen atom, halogen, or methyl,
   or a pharmaceutically acceptable complex thereof.
(303) Use of a compound according to (301) or (302) above or a pharmaceutically acceptable complex thereof for the production of a pharmaceutical composition for the treatment and/or prevention of a disorder in social communication and/or reciprocal social interaction.
(304) Use of the compound according to any one of (301) to (303) above or a pharmaceutically acceptable complex thereof, for production of a pharmaceutical composition for treatment and/or prevention of autism spectrum disorder, fragile X syndrome, and/or autism spectrum disorder-like symptoms.
(305) Use of a compound according to any one of (301) to (304) or a pharmaceutically acceptable complex thereof, for producing a pharmaceutical composition for treatment and/or prevention of autism spectrum disorder.
(306) The use according to any one of (301) to (305) above, wherein
   the compound represented by the Formula (I) or a pharmaceutically acceptable complex thereof is the following compound: 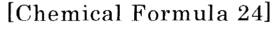 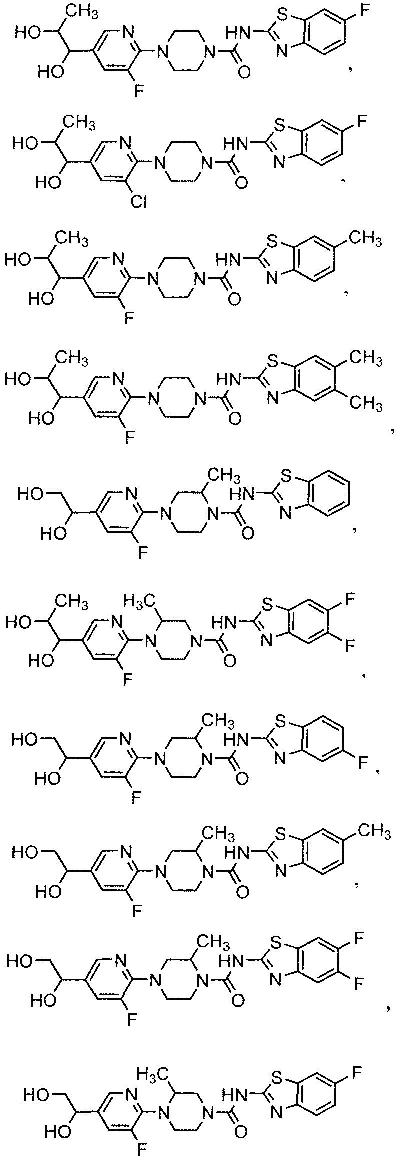 or
   or a pharmaceutically acceptable complex thereof.
(306A) The use according to any one of (201) to (306) above, wherein
   the compound represented by the Formula (I) or a pharmaceutically acceptable complex thereof is the following compound: 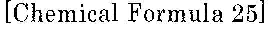 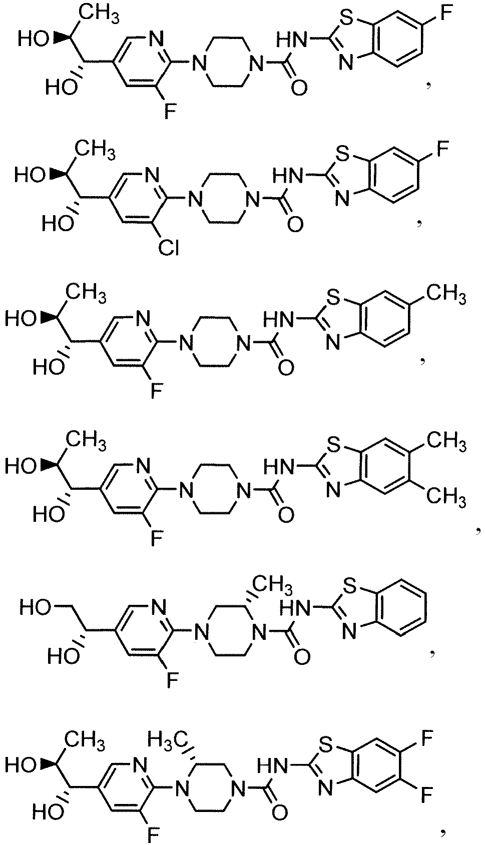 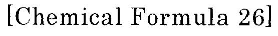 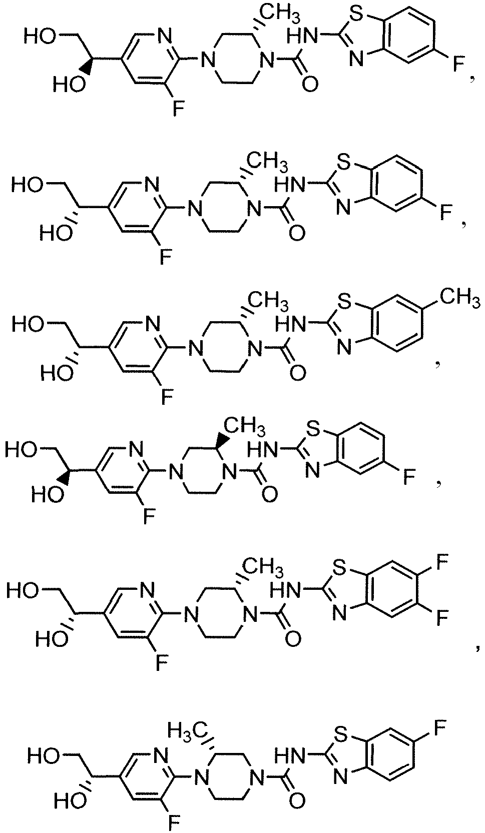 or
   or a pharmaceutically acceptable complex thereof.
(307) The use according to any one of (301) to (306) above, wherein
   the compound represented by the Formula (I) or a pharmaceutically acceptable complex thereof is a compound represented by Formula (I-A): 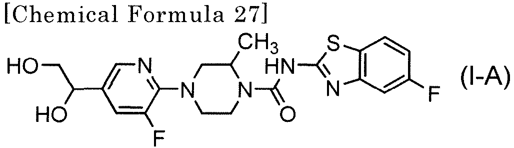
   or a pharmaceutically acceptable complex thereof.
(308) The use according to any one of (301) to (307) above, wherein
   the compound represented by the Formula (I) or a pharmaceutically acceptable complex thereof is a compound represented by Formula (I-B): 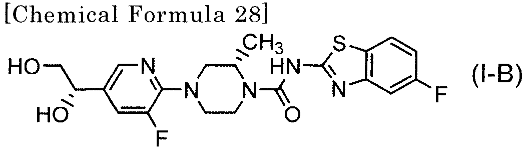
   or a pharmaceutically acceptable complex thereof.
(309) The use according to any one of (301) to (308) and (306A) above, wherein the pharmaceutically acceptable complex is a complex with hydrochloric acid, tartaric acid, benzenesulfonic acid, p-toluenesulfonic acid, or fumaric acid.
(310) The use according to any one of (301) to (309) and (306A) above, wherein the pharmaceutically acceptable complex is a complex with fumaric acid.
(311) The use according to any one of (301) to (310) and (306A) above, wherein the pharmaceutically acceptable complex is a fumaric acid cocrystal.
(312) The use according to any one of (301) to (311) and (306A) above, wherein the pharmaceutically acceptable complex is a complex in which the molar ratio of the compound represented by the Formula (I) and fumaric acid is about 1 : about 0.5 (or about 2 : about 1).
(313) The use according to any one of (301) to (312) and (306A) above, wherein the pharmaceutically acceptable complex is a complex in which the molar ratio of the compound represented by the Formula (I) and fumaric acid is 1 : 0.5 ± 0.2.
(314) The use according to any one of (301) to (313) and (306 A), wherein a dose of the compound represented by the formula (I) or the pharmaceutically acceptable complex thereof is 1 mg to 1000 mg per administration.
(315) The use according to any one of (301) to (314) and (306A) above, for treating and/or preventing a core symptom of autism spectrum disorder.

### [EFFECT OF THE INVENTION]

The pharmaceutical compositions and methods of the present invention are useful for the treatment and/or prevention of various psychiatric diseases, in particular social impairments, such as autism spectrum disorder, fragile X syndrome, autism spectrum disorder-like symptoms, and/or disorders in social communication and/or reciprocal social interaction.

### [BRIEF DESCRIPTION OF THE DRAWINGS]

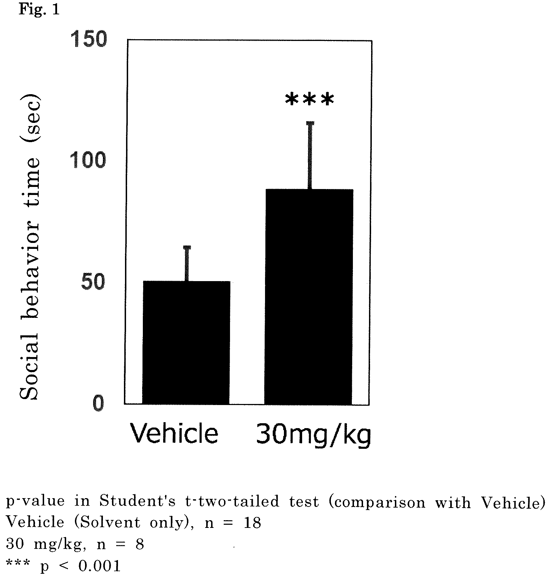

Fig. 1 shows the results of evaluating the social ameliorating effect of a compound represented by Formula (I-B) using a BTBR model mouse which is an ASD model mouse.

### [MODE FOR CARRYING OUT THE INVENTION]

The term "consisting of" means having only the components. The term "comprising" means being not limited to the components, but not excluding elements that are not described.

Hereinafter, the present invention will be described with reference to embodiments. Throughout the present specification, an expression in a singular form should be understood as also including the concept of its plural form, unless otherwise stated. Therefore, singular articles (for example, "a", "an", "the", and the like in English) should be understood as also including the concept of their plural form, unless otherwise stated.

In addition, the terms used in the present specification should be understood as being used in the meanings commonly used in the art unless otherwise stated. Therefore, unless otherwise defined, all terminology and scientific terms used in the present specification have the same meanings as commonly understood by those skilled in the art to which the present invention belongs. In case of conflict, the present specification (including definitions) prevails.

The term "halogen" includes a fluorine atom, a chlorine atom, a bromine atom and an iodine atom. A fluorine atom and a chlorine atom are preferable, and a fluorine atom is particularly preferable.

The term "alkyl" includes a C1 to C15, preferably a C1 to C10, more preferably a C1 to C6, and further preferably a C1 to C4 linear or branched hydrocarbon group. Examples include methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl and n-decyl.

Preferred embodiments of "alkyl" include methyl, ethyl, n-propyl, isopropyl, n-butyl, isobutyl, sec-butyl, tert-butyl and n-pentyl. A more preferred embodiment is methyl, ethyl, n-propyl, isopropyl or tert-butyl.

Examples of the substituent of "substituted or unsubstituted alkyl", "substituted or unsubstituted alkyloxy", and "substituted or unsubstituted alkyloxycarbonyl" include substituent group α.

Substituent group α: halogen, hydroxy, carboxy, alkyloxy, haloalkyloxy, alkenyloxy, alkynyloxy, alkylcarbonyl, haloalkylcarbonyl, alkenylcarbonyl, alkynylcarbonyl, sulfanyl, and cyano.

The "social impairment" includes, for example, disorders in social communication and/or reciprocal social interaction, including, for example, autism spectrum disorder, fragile X syndrome, and autism spectrum disorder-like symptoms, such as autism spectrum disorder.

The "Autism Spectrum Disorder (ASD)" is a type of neurodevelopmental disorder (neurodevelopmental disorder group), and includes autism disorder, Asperger's disorder, and pervasive developmental disorder not otherwise specified. For the term (definition), characteristics of a disorder, and diagnostic criteria, "International Classification of Diseases (ICD) 11th edition" prepared by the World Health Organization (WHO), "DSM-5 (registered trademark) Manual of Diagnosis and Statistics of Mental Diseases" prepared by the American Psychiatric Association, and the like can be referred to. Note that the criteria are not particularly limited to the ICD-11 and the DSM-5 as long as criteria that can be used to accurately diagnose autism spectrum disorder are acceptable.

Core symptoms of autism spectrum disorder include "persistent disorders in social communication and/or reciprocal social interaction" and "symptoms related to restrictive and repetitive patterns of behaviors, interests, and activities" in patients with autism spectrum disorder, e.g., persistent disorders in social communication and/or reciprocal social interaction.

The "autism spectrum disorder-like symptoms" include a case in which the diagnostic criteria for autism spectrum disorder are not satisfied, but autism spectrum disorder-like symptoms are exhibited. The "autism spectrum disorder-like symptoms" further include autism spectrum disorder-like symptoms associated with other diseases (For example, fragile X syndrome, Rett syndrome, tuberous sclerosis (TSC), etc.). The "autism spectrum disorder-like symptoms" include, for example, "(persistent) disorder in social communication and/or reciprocal social interaction" and "symptoms related to restrictive and repetitive patterns of behaviors, interests, and activities", and are preferably (persistent) disorder in social communication and/or reciprocal social interaction.

For the terms (definitions) of "fragile X syndrome" and "Rett syndrome", characteristics of the disorder, and diagnostic criteria, for example, "International Classification of Diseases (ICD) 11th edition" prepared by the World Health Organization (WHO), can be referred to.

In this specification, unless the context dictates otherwise, reciting "about" before a numerical value X includes the range of +/-10% of the numerical value X and includes the numerical value that, having regard to the significant figure of the numerical value X, is rounded to the nearest significant figure.

For example, about 1 mg includes 0.9 mg and 1.4 mg.

A preferred embodiment of each substituent in the compound represented by the formula (I) is described below. Examples of the compound represented by Formula (I) include embodiments of all combinations of specific examples described below.
R¹ is substituted or unsubstituted alkyl.
R¹ is alkyl may be substituted with one or more groups selected from substituent group α.
R¹ is alkyl substituted with one or more hydroxy.
R¹ is C2-C3 alkyl substituted with one or more hydroxy.
R¹ is C2-C3 alkyl substituted with two hydroxy.
R² is a hydrogen atom, halogen, or substituted or unsubstituted alkyl.
R² is a hydrogen atom, halogen, or alkyl may be substituted with one or more halogens.
R² is halogen.
A is N, CH or C.
A is N.
A is CH.
A is C. 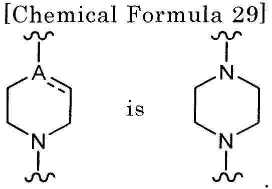
R³ is substituted or unsubstituted alkyl or halogen.
R³ is substituted or unsubstituted alkyl.
R³ is alkyl may be substituted with one or more groups selected from substituent group α.
R³ is C1 to C3 alkyl may be substituted with one or more groups selected from substituent group α.
R³ is methyl.
m is 0 or 1.
m is 1.
m is 0.

R⁴ and R⁵ are each independently a hydrogen atom, halogen, substituted or unsubstituted alkyl, substituted or unsubstituted alkyloxy, or substituted or unsubstituted alkyloxycarbonyl.

R⁴ and R⁵ are each independently an alkyl may be substituted with one or more groups selected from substituent group α, an alkyloxy may be substituted with one or more groups selected from substituent group α, an alkyloxycarbonyl may be substituted with one or more groups selected from substituent group α, a hydrogen atom, or a halogen.

R⁴ and R⁵ are each independently C1 to C3 alkyl may be substituted with one or more groups selected from substituent group α, a hydrogen atom, or halogen.

R⁴ and R⁵ are each independently a hydrogen atom, halogen, or methyl.

The group represented by [Chemical Formula 30] 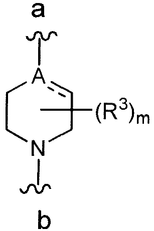 (wherein bond a is bonded to the pyridine ring and bond b is bonded to the carbonyl group.) includes the following aspects. When A is CH, the H may be substituted with R³.

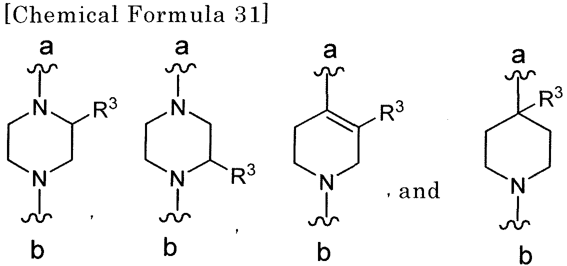

Preferably, the group represented by 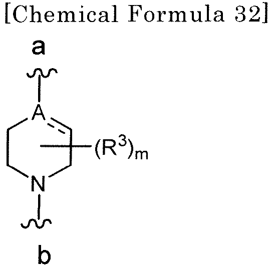 is

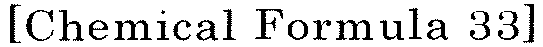

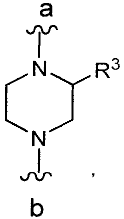

A "pharmaceutically acceptable complex" includes a pharmaceutically acceptable salt, a pharmaceutically acceptable cocrystal, and a combination thereof. Preferably, it is a pharmaceutically acceptable cocrystal.

The "pharmaceutically acceptable complex" is preferably a complex with hydrochloric acid, tartaric acid, benzenesulfonic acid, p-toluenesulfonic acid, or fumaric acid. Particularly preferred is a complex with fumaric acid.

For example, "complex with fumaric acid" includes fumarate, a cocrystal with fumaric acid, or a combination thereof.

In the present description, as the "pharmaceutically acceptable salt", examples of basic salts include alkali metal salts such as lithium salt, sodium salt and potassium salt; alkaline earth metal salts such as calcium salt and barium salt; transition metal salts such as zinc salt and iron salt; magnesium salt; ammonium salt; aliphatic amine salts such as trimethylamine salt, triethylamine salt, dicyclohexylamine salt, ethanolamine salt, diethanolamine salt, triethanolamine salt, ethylenediamine salt, meglumine salt and procaine salt; aralkylamine salts such as N,N-dibenzylethylenediamine; aromatic heterocyclic amine salts such as pyridine salt, picoline salt, quinoline salt and isoquinoline salt; quaternary ammonium salts such as tetramethylammonium salt, tetraethylammonium salt, benzyltrimethylammonium salt, benzyltriethylammonium salt, benzyltributylammonium salt, methyltrioctylammonium salt and tetrabutylammonium salt; and basic amino acid salts such as arginine salt and lysine salt. Examples of acidic salts include inorganic acid salts such as hydrochloride, sulfate, nitrate, phosphate, carbonate, hydrogen carbonate, hydrobromate, hydroiodide and perchlorate; organic acid salts such as formate, acetate, propionate, trifluoroacetate, citrate, lactate, tartrate, oxalate, maleate, fumarate, succinate, mandelate, glutarate, malate, benzoate, phthalate and ascorbate; sulfonates such as methanesulfonate, ethanesulfonate, isethionate, benzenesulfonate and p-toluenesulfonate; and acidic amino acid salts such as aspartate and glutamate. These salts can be formed by a conventional method. In other embodiments, the pharmaceutically acceptable salt is fumarate. In other embodiments, the pharmaceutically acceptable fumarate salt contains about 1 equivalent of the compound of Formula (I) and about 0.5 equivalents of fumaric acid, such as from about 0.3 to about 0.7 equivalents of fumaric acid in one embodiment, from about 0.4 to about 0.6 equivalents of fumaric acid in other embodiments, from about 0.44 to about 0.56 equivalents of fumaric acid in other embodiments, or from about 0.47 to about 0.53 equivalents of fumaric acid in other embodiments. In other embodiments, the pharmaceutically acceptable fumarate contains 1 equivalent of the compound of Formula (I) and 0.5 equivalents of fumaric acid. In one aspect, it contains about 2 equivalents of a compound of Formula (I) and about 1 equivalent of fumaric acid. The person skilled in the art can prepare the acid addition salts of the compounds of the Formula (I), for example by reacting the compound with a suitable acid in a variety of known ways.

Examples of the "pharmaceutical acceptable cocrystal" of the compound represented by Formula (I) (including the compound represented by Formula (I-A) and the compound represented by Formula (I-B), the same applies below) include inorganic acids such as hydrochloride, sulfuric acid, nitric acid, phosphoric acid, carbonic acid, hydrogen carbonate, hydrobromic acid, hydroiodic acid, and perchloric acid.; organic acids such as formic acid, acetic acid, propionic acid, trifluoroacetic acid, citric acid, lactic acid, tartaric acid, oxalic acid, maleic acid, fumaric acid, succinic acid, mandelic acid, glutaric acid, malic acid, benzoic acid, phthalic acid, and ascorbic acid; sulfonic acids such as methanesulfonic acid, ethanesulfonic acid, isethionic acid, benzenesulfonic acid, and p-toluenesulfonic acid; cocrystals with acidic amino acids such as aspartic acid and glutamic acid. Another aspect includes cocrystals with hydrochloric acid, tartaric acid, benzenesulfonic acid, toluenesulfonic acid, succinic acid, fumaric acid, citric acid, oxalic acid, benzoic acid, cinnamic acid, caproic acid, sorbic acid, and the like.

In one embodiment, the cocrystal with the compound of Formula (I) includes hydrochloric acid, tartaric acid, benzenesulfonic acid, toluenesulfonic acid, succinic acid, fumaric acid, citric acid, oxalic acid, benzoic acid, or any mixture thereof. In other embodiments, the cocrystal with a compound of Formula (I) includes hydrochloric acid, benzenesulfonic acid, toluenesulfonic acid, L-tartaric acid, fumaric acid, or any mixture thereof. In other embodiments, the cocrystal consists of a compound of Formula (I) and hydrochloric acid. In other embodiments, the cocrystal consists of a compound of Formula (I) and benzenesulfonic acid. In other embodiments, the cocrystal consists of a compound of Formula (I) and toluenesulfonic acid. In other embodiments, the cocrystal consists of a compound of Formula (I) and L-tartaric acid. In other embodiments, the cocrystal consists of a compound of Formula (I) and fumaric acid. In other embodiments, the cocrystal contains about 1 equivalent of a compound of Formula (I) and about 0.5 equivalents of fumaric acid, such as from about 0.3 to about 0.7 equivalents of fumaric acid in one embodiment, from about 0.4 to about 0.6 equivalents of fumaric acid in other embodiments, from about 0.44 to about 0.56 equivalents of fumaric acid in other embodiments, or from about 0.47 to about 0.53 equivalents of fumaric acid in other embodiments. In other embodiments, the cocrystal contains 1 equivalent of a compound of Formula (I) and 0.5 equivalents of fumaric acid. In one aspect, it contains about 2 equivalents of a compound of Formula (I) and about 1 equivalent of fumaric acid.

However, it is known in the art that "The exact classification of a compound as a salt or cocrystal can sometimes be somewhat ambiguous" (Aakeroy et al., "Cocrystal or Salt: Does it really matter?", Mol Pharmaceutics 4 (3): 317 to 322 (2007)). when the phrase "and combinations thereof" is used in the context of a salt and/or a cocrystal, in one embodiment the properties ascribed to the salt and other properties ascribed to the cocrystal are simultaneously present; in other embodiments, it should be understood to mean that there are intermediate properties between those attributed to the salt and those attributed to the cocrystal.

The compound represented by the Formula (I) or the pharmaceutically acceptable complex thereof may be a solvate, a cocrystal and/or a crystal polymorph.

Solvates include organic solvates in which any number of organic solvent molecules are coordinated and hydrates in which any number of water molecules are coordinated. In the present description, the term "solvate" means a solvate of the compound or a pharmaceutically acceptable salt thereof, and examples thereof include a monosolvate, a disolvate, a monohydrate and a dihydrate. Examples thereof include a hydrate, an ethanol hydrate, a methyl acetate hydrate, an ethyl acetate and a 2-propanol hydrate, an n-propyl acetate and a 2-propanol hydrate, an acetonitrile hydrate, a 1,2-dimethoxyethane hydrate, and a methyl isobutyl ketone hydrate, and preferably include a hydrate such as a monohydrate, for example, trihydrate.

The compound represented by Formula (I) or a pharmaceutically acceptable complex thereof may be administered as a possible isomer of the compound represented by Formula (I) (for example, a keto-enol isomer, an imine-enamine isomer, a diastereoisomer, an optical isomer, a rotational isomer, a racemate, or a mixture thereof), a prodrug, or a pharmaceutically acceptable complex thereof.

The compound represented by the Formula (I) or a pharmaceutically acceptable complex thereof may form a prodrug, and the present invention also includes various such prodrugs. Prodrugs are derivatives of the compounds according to the present invention that have chemically or metabolically degradable groups, and compounds that are converted to the pharmaceutically active compounds according to the present invention through solvolysis or under physiological conditions in vivo. Prodrugs include compounds that are converted to the compounds represented by Formula (I) through enzymatic oxidation, reduction, hydrolysis or the like under physiological conditions and in vivo, compounds that are converted to the compounds represented by Formula (I) through hydrolysis by gastric acid etc., and the like. Methods for selecting and preparing suitable prodrug derivatives are described in, for example, "Design of Prodrugs, Elsevier, Amsterdam, 1985". Prodrugs themselves may have some activity.

One or more hydrogen, carbon and/or other atoms of the compound represented by the Formula (I) may be substituted by isotopes of hydrogen, carbon and/or other atoms, respectively. Examples of such an isotope include hydrogen, carbon, nitrogen, oxygen, phosphorus, sulfur, fluorine, iodine, and chlorine such as ²H, ³H, ¹¹C, ¹³C, ¹⁴C, ¹⁵N, ¹⁸O, ¹⁷O, ³¹P, ³²P, ³⁵S, ¹⁸F, ¹²³O, and ³⁶Cl. The compound represented by Formula (I) also includes a compound substituted with such an isotope. The compound substituted with the isotope is also useful as a pharmaceutical product and includes all radiolabeled forms of the compound represented by Formula (I). Furthermore, a "radiolabeling method" for production of the "radiolabeled form" is also included in the present invention, and the "radiolabeled form" is useful as a tool for metabolic pharmacokinetic studies and for research and/or diagnosis in binding assay.

The radiolabeled form of the compound represented by the Formula (I) can be prepared by a method well known in the art. For example, a tritium-labeled compound represented by Formula (I) can be prepared by introducing tritium into a specific compound represented by Formula (I) by catalytic dehalogenation reaction using tritium. This method includes reacting an appropriately halogenated precursor of the compound represented by Formula (I) with tritium gas in the presence of an appropriate catalyst, such as Pd/C, and in the presence or absence of a base. Regarding other appropriate methods for preparing tritium-labeled compounds, "Isotopes in the Physical and Biomedical Sciences, Vol. 1, Labeled Compounds (Part A), Chapter 6 (1987)" can be referred to. A ¹⁴C-labeled compound can be prepared by using a raw material having ¹⁴C carbon.

A pharmaceutical composition can be obtained by mixing an effective amount of the compound used in the present invention with various pharmaceutical additives appropriate for the dosage form, such as an excipient, a binder, a disintegrating agent, and a lubricating agent, as necessary. Furthermore, the pharmaceutical composition can be prepared into a pharmaceutical composition for use for a child, an elderly, a patient with a serious case, or a surgical operation, by appropriately changing the effective amount of the compound, the dosage form, and/or various pharmaceutical additives.

The administration route of the pharmaceutical composition of the present invention is not particularly limited, and the pharmaceutical composition can be administered orally or parenterally. Examples of a method of parenteral administration include transdermal, subcutaneous, intravenous, intraarterial, intramuscular, intraperitoneal, transmucosal, inhalation, transnasal, ophthalmic, inner ear, or vaginal administration.

In the case of oral administration, the pharmaceutical composition may be prepared into any dosage form that is commonly used, such as a solid formulation for internal use (e.g., a tablet, a powder, a granule, a capsule, a pill, a film, or the like) or a liquid formulation for internal use (e.g., a suspension, an emulsion, an elixir, a syrup, a lemonade, a spirit, an aromatic water, an extract, a decoction, a tincture, or the like), and administered. The tablet may be a sugar-coated tablet, a film-coated tablet, an enteric-coated tablet, a sustained-release tablet, a troche tablet, a sublingual tablet, a buccal tablet, a chewable tablet, or an orally disintegrating tablet; the powder and the granule may be a dry syrup; and the capsule may be a soft capsule, a micro capsule, or a sustained-release capsule.

In the case of parenteral administration, the pharmaceutical composition can be suitably administered in any dosage form that is commonly used, such as an injection, an infusion, or a preparation for external use (e.g., an eye drop, a nasal drop, an ear drop, an aerosol, an inhalant, a lotion, an impregnating agent, a liniment, a gargling agent, an enema, an ointment, a plaster, a jelly, a cream, a patch, a poultice, a powder for external use, a suppository, or the like). The injection may be an emulsion of O/W, W/O, O/W/O, W/O/W type, or the like.

The pharmaceutical composition for transdermal administration is not particularly limited, and examples thereof include an external solid agent (for example, external powders), an external liquid agent (for example, a liniment agent and a lotion agent), spray (an external aerosol, pump spray), ointment, cream, gel, and a patch (for example, a tape agent (for example, a plaster agent, plaster) or a cataplasm agent).

An aspect of the method, the agent, and the pharmaceutical composition of the present invention is not particularly limited, and can be appropriately determined according to the degree of a symptom, the body weight, the age, the administration form of the agent, and the like of the patient.

A pharmaceutical composition can be obtained by mixing an effective amount of the compound of the present invention with various pharmaceutical additives appropriate for the dosage form, such as an excipient, a binder, a disintegrating agent, and a lubricating agent, as necessary. Furthermore, the pharmaceutical composition can also be a pharmaceutical composition for children, elderly people, and severe patients by appropriately changing the effective amount, dosage form, and/or various pharmaceutical additives of the compound of the present invention. For example, pediatric pharmaceutical compositions may be administered to patients who are neonates (younger than 4 weeks old after the birth), infants (4 weeks old to younger than 1 year old after the birth), children (1 year old or older and younger than 7 years old), infant children (7 years old or older and younger than 15 years old), or 15 to 18 years old. For example, the geriatric pharmaceutical compositions may be administered to patients who are 65 years old or older.

The dose of the compound represented by Formula (I) or the pharmaceutically acceptable complex thereof in the pharmaceutical composition of the present invention is desirably set in consideration of the age, body weight, type and degree of disease of the patient, administration route, and the like.

The dose per one time is, for example, 0.1 mg to 2000 mg, for example, 0.1 mg to 1000 mg, for example, 1 mg to 1000 mg, for example, 1 mg to 600 mg, preferably 1 mg to 300 mg, for example, 1 mg to 200 mg, for example, 1 mg to 100 mg. For example, it can be administered once a day. For example, it can be administered twice a day.

The daily dose is, for example, 0.1 mg to 2000 mg, for example, 0.1 mg to 1000 mg, for example, 1 mg to 600 mg, preferably 1 mg to 300 mg, for example, 1 mg to 200 mg, for example, 1 mg to 100 mg.

For example, it can be orally administered at the above dosages.

As another aspect, the dose of the compound represented by Formula (I) or the pharmaceutically acceptable complex thereof in the pharmaceutical composition of the present invention is, for example, 0.1 mg/kg to 1000 mg/kg, for example, 0.1 mg/kg to 500 mg/kg, for example, 0.1 mg/kg to 100 mg/kg, 0.1 mg/kg to 30 mg/kg, for example, 1 mg/kg to 30 mg/kg, for example, 1 mg/kg to 10 mg/kg, for example, 1 mg/kg to 5 mg/kg, for example, 2.5 mg/kg to 5 mg/kg.

In the case of parenteral administration, the dose varies greatly depending on the administration route, but is, for example, 0.005 to 10 mg/kg/day, preferably 0.01 to 1 mg/kg/day. The dosage may be administered in one to several divisions per day.

The method and pharmaceutical composition, etc. of the present invention can include attachment and label which are described for providing instructions to paramedical personnel undertaking prevention or treatment such as physicians, for specification of a patient or a subject targeted by the present invention as a therapeutic objective, and guidelines for treatment and/or prevention such as dosage and administration, and precautions. However, these public documents are not limited to paper media, but can be provided through the Internet, and guidelines for prevention or treatment can be provided to physicians and the like on the basis of various other information sources in addition to public documents. Therefore, it should be understood that the present invention also includes embodiments that are used on the basis of information other than attachment and label.

As aspects of the method, agent, pharmaceutical preparation, and pharmaceutical composition of the present invention, they may be used in combination with other psychiatric drugs (including digital drugs) and/or other therapies (including various treatment methods without a drug), or may be used without these.

The pharmaceutical composition or method of the present invention preferably has one or more superior characteristics selected from the following.
a) It is useful in the treatment and/or prevention of social impairments, such as autism spectrum disorder, fragile X syndrome, autism spectrum disorder-like symptoms, and/or disorders in social communication and/or reciprocal interactions. Preferably, it is useful for treatment and/or prevention of autism spectrum disorder. Particularly preferably, it is useful for the treatment and/or prevention of a core symptom of autism spectrum disorder.
b) High safety. For example, it can be a pharmaceutical product having a low effective drug efficacy amount and concentration and a high safety margin. In addition, it may be a pharmaceutical product having a low risk of a side action. For example, the risk of side effects of increased body temperature is low. For example, the risk of central side effects is low due to low brain transferability.

The compound represented by the Formula (I) or a pharmaceutically acceptable complex thereof can be synthesized according to a known method. For example, Patent Documents 1 to 5 can be referred to.

Patent Document 2 describes a method for preparing a compound represented by Formula (I) of the present application or a pharmaceutically acceptable complex thereof, a result of a binding assay with TRPV1, and the like. For example, Example 12 of Patent Document 2 describes the results of a TRPV1 binding assay of a compound represented by Formula (I) of the present application (For example, compounds L1, R1, W1, V2, W2, B3, P3, C4, H4, L4, Z4, F5, N5, O5, Z5, V6 and the like in Patent Document 2) and the like.

Patent Document 3 describes a method for preparing a compound represented by Formula (I) of the present application or a pharmaceutically acceptable complex thereof, a result of a binding assay with TRPV1, and the like. For example, in Table 4 of Patent Document 3, assay results of compounds represented by Formula (I) of the present application (For example, compounds ACU, ABD, ADF, and AAH in Patent Document 3) and the like are described. For example, in Table 5 of Patent Document 3, assay results of compounds represented by Formula (I) of the present application (For example, compounds AEF and ADK in Patent Document 3) and the like are described.

Patent Document 5 describes a method for preparing a compound represented by Formula (I) or a pharmaceutically acceptable complex thereof, and test results of a compound represented by Formula (I) or a pharmaceutically acceptable complex thereof. For example, Example 10 of Patent Document 5 describes a method for preparing a complex having an average molar ratio of a compound represented by Formula (I-B) of the present application (compound A155 (a) of Patent Document 5) and fumaric acid of about 1 : 0.5 ± 0.2, and powder X-ray analysis results, and the like. In addition, it is described that the complex showed cocrystalline properties from NMR analysis and the like. Furthermore, Fig. 2 and the like of Patent Document 5 describe a dihydrochloride of a compound represented by Formula (I-B) of the present application (Compound A155 (a) of Patent Document 5). Examples 11, 12, 13, and 14 each describe a combination of a compound represented by Formula (I-B) of the present application (compound A155 (a) in Patent Document 5) and hydrochloric acid, tartaric acid, benzenesulfonic acid, or toluenesulfonic acid. In addition, Example 15 describes the test results (binding assay with TRPV1) of a compound represented by Formula (I) (a large number of compounds A122 (a), A122 (b), A122 (c), A122 (e), A123 (e), A125 (b), A125 (e), A126 (a), A126 (e), A155 (a), A155 (b), A155 (d), A155 (e), A158 (a), B122 (j), B122 (k), and the like) of the present application. Example 17 describes in vivo assay results of hyperthermia.

### [EXAMPLES]

Hereinafter, the present invention will be described based on examples. However, the present invention is not limited to these examples and the like.

### (Example 1: Assessment of drug efficacy using BTBR mice)

BTBR T + tf/J (BTBR) mice are one of the popular ASD model mice, as ASD-like symptoms such as the social impairment and a repetitive behavior consistent with diagnostic criteria for ASD are observed (Physiol Behav. 2012 Dec 5; 107 (5): 649-662. Doi: 10.1016/j.physbeh. 2011. 12. 025). This mouse was used to evaluate the efficacy of the compound represented by the Formula (I-B) against the social impairment. As a social evaluation test, a reciprocal social interaction test was used. In this test, two mice are placed in one cage, and the time (seconds) the subject mouse (Test mouse) performs sniffing, which is a social behavior, to another mouse(Intruder mouse) is quantified as an index of social behavior.

As a control group, a 0.5w/v% methyl cellulose 400 solution (vehicle) or a fumaric acid cocrystal of a compound represented by Formula (I-B) (a complex in which the molar ratio of the compound represented by Formula (I-B) and fumaric acid was about 1 : about 0.5) (30 mg/kg) was orally administered, and the amount of social behavior for 5 minutes from 1 hour after administration was quantified. The fumaric acid cocrystal of the compound represented by Formula (I-B) at 30 mg/kg is 26.57 mg/kg as a compound-free form represented by Formula (I-B).

The results are shown in Fig. 1. Compared with the vehicle, the administration of 30 mg/kg of the fumaric acid cocrystal of the compound represented by the Formula (I-B) significantly increased the amount of social behavior.

From the above results, it is considered that the compound represented by Formula (I-B) or a pharmaceutically acceptable complex thereof has an improvement action of social impairment in ASD and is useful as a therapeutic agent for ASD.

Similarly, a compound represented by Formula (I-B) or a pharmaceutically acceptable complex thereof (For example, a fumaric acid cocrystal of a compound represented by Formula (I-B)) can be administered to a BTBR model mouse at 1 mg/kg, 3 mg/kg, or 10 mg/kg, respectively, to evaluate the improvement action of social impairment.

### (Formulation Example)

The following Formulation Examples are merely examples and not intended to limit the scope of the invention.

A compound represented by Formula (I) or a pharmaceutically acceptable complex thereof can be administered as a pharmaceutical composition by any conventional route, for example, orally, for example, in the form of a tablet or capsule, or parenterally, for example, in the form of an injection solution or a suspension, topically, for example, in the form of a lotion, a gel, an ointment or a cream, or in the form of a nasal form or a suppository. A pharmaceutical composition including the compound represented by Formula (I) in free form or in the form of a pharmaceutically acceptable complex or a pharmaceutically acceptable complex thereof together with at least one pharmaceutically acceptable carrier or diluent can be produced in a conventional manner by mixing, granulating or coating methods. For example, the oral composition can be a tablet, a granule, or a capsule, each containing an excipient, a disintegrant, a binder, a lubricant, and the like, as well as an active ingredient and the like. Furthermore, the injectable composition can be a solution or a suspension, may be sterilized, and may contain a preservative, a stabilizer, a buffering agent, and the like.

### [INDUSTRIAL APPLICABILITY]

The pharmaceutical composition of the present invention including a compound represented by Formula (I) or a pharmaceutically acceptable complex thereof and the method for administering the compound represented by the Formula (I) or a pharmaceutically acceptable complex thereof are useful for the treatment and/or prevention of social impairments, such as autism spectrum disorder, fragile X syndrome, and/or autism spectrum disorder-like symptoms.

## Claims

1. A pharmaceutical composition for treatment and/or prevention of social impairment, comprising a compound represented by Formula (I): 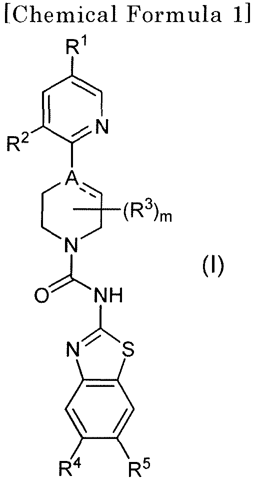 wherein
R¹ is substituted or unsubstituted alkyl,
R² is a hydrogen atom, halogen, or substituted or unsubstituted alkyl,
A is N, CH, or C,
when A is N or CH, a dashed line indicates absence of a bond,
when A is C, the dashed line indicates presence of a bond,
R³ is substituted or unsubstituted alkyl or halogen,
m is 0 or 1, and
R⁴ and R⁵ are each independently a hydrogen atom, halogen, substituted or unsubstituted alkyl, substituted or unsubstituted alkyloxy, or substituted or unsubstituted alkyloxycarbonyl,
or a pharmaceutically acceptable complex thereof.

2. The pharmaceutical composition according to claim 1, wherein
R¹ is C2-C3 alkyl substituted with one or more hydroxy,
R² is halogen, 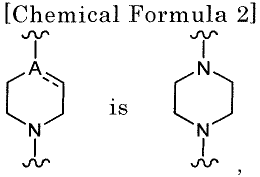
R³ is methyl, and
R⁴ and R⁵ are each independently a hydrogen atom, halogen, or methyl.

3. The pharmaceutical composition according to claim 1 or 2, wherein the social impairment is a disorder in social communication and/or reciprocal social interaction.

4. The pharmaceutical composition according to claim 1 or 2, wherein the social impairment is autism spectrum disorder, fragile X syndrome, and/or autism spectrum disorder-like symptoms.

5. The pharmaceutical composition according to claim 1 or 2, wherein the social impairment is autism spectrum disorder.

6. The pharmaceutical composition according to any one of claims 1 to 5, wherein
the compound represented by the Formula (I) or a pharmaceutically acceptable complex thereof is the following compound: 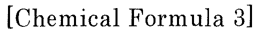 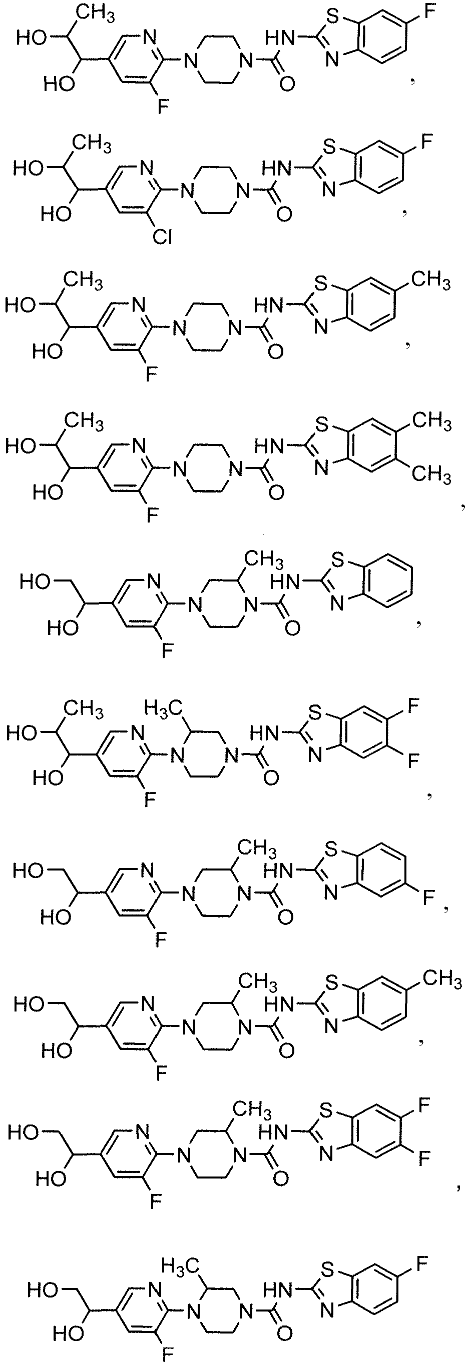 or
or a pharmaceutically acceptable complex thereof.

7. The pharmaceutical composition according to any one of claims 1 to 5, wherein
the compound represented by the Formula (I) or a pharmaceutically acceptable complex thereof is a compound represented by Formula (I-A): 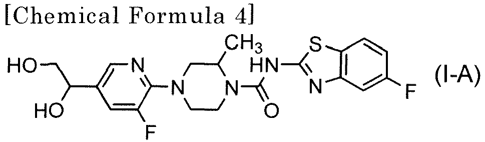
or a pharmaceutically acceptable complex thereof.

8. The pharmaceutical composition according to any one of claims 1 to 5, wherein
a compound represented by the Formula (I) or a pharmaceutically acceptable complex thereof is a compound represented by Formula (I-B): 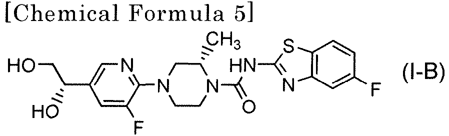
or a pharmaceutically acceptable complex thereof.

9. The pharmaceutical composition according to any one of claims 1 to 8, wherein the pharmaceutically acceptable complex is a complex with hydrochloric acid, tartaric acid, benzenesulfonic acid, p-toluenesulfonic acid, or fumaric acid.

10. The pharmaceutical composition according to any one of claims 1 to 9, wherein the pharmaceutically acceptable complex is a complex with fumaric acid.
